# EUROPEAN PATENT APPLICATION

(11) **EP 4 674 851 A1**
(43) Date of publication of application: **07.01.2026**
(21) Application number: 24763244.1
(22) Date of filing: 01.03.2024
(51) Int. Cl.: C07D 491/22, C07K 5/062, A61K 39/395

(54) **PREPARATION METHOD FOR INDOLIZINO[1,2-B]QUINOLINE-10,13-DIONE DERIVATIVE**

(30) Priority: 01.03.2023 CN 202310186630
(71) Applicant: Jiangsu Hengrui Pharmaceuticals Co., Ltd., Lianyungang, Jiangsu 222047 (CN)
(72) Inventor: ZHU, Changzhen, Lianyungang, Jiangsu 222047 (CN); MEI, Jie, Lianyungang, Jiangsu 222047 (CN); ZHANG, Lei, Lianyungang, Jiangsu 222047 (CN); LI, Tongguo, Lianyungang, Jiangsu 222047 (CN)
(74) Representative: Dragotti & Associati S.P.A.
(86) International application number: PCT/CN2024/079531
(87) International publication number: WO 2024/179564

(57) **Abstract**

The present disclosure relates to a method for preparing an indolizino[1,2-b]quinoline-10,13-dione derivative. Specifically, the present disclosure relates to a method for preparing a compound of formula I. The method comprises the step of using a compound of formula D-a to form the compound of formula I under acidic conditions. The method can efficiently achieve the enrichment of the compound of formula I and increase process yield, and is suitable for industrial production.

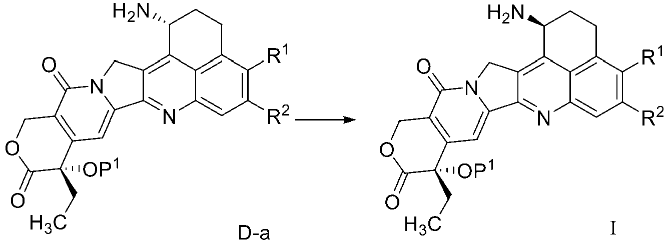

## Description

### TECHNICAL FIELD

The present disclosure pertains to the field of pharmaceutics and relates to a preparation method for an indeno[1,2-b]quinoline-10,13-dione derivative.

### BACKGROUND

The compound (1S,9S)-1-amino-9-ethyl-5-fluoro-2,3-dihydro-9-hydroxy-4-methyl-1H,12H-benzo[de]pyrano[3',4':6,7]imidazo[1,2-b]quinoline-10,13(9H,15H)-dione is a camptothecin derivative. It is a topoisomerase I, plays a role in inhibiting tumor proliferation, and has an antitumor effect.

EP0495432 discloses a preparation method for the aforementioned compound. The method mainly uses compound 1 as a substrate. The substrate undergoes a Friedlander reaction with compound 2 to form intermediate 3. Subsequently, the acetyl group is removed under methanesulfonic acid conditions, yielding diastereomers. The diastereomers are then separated by recrystallization to give the optical product:

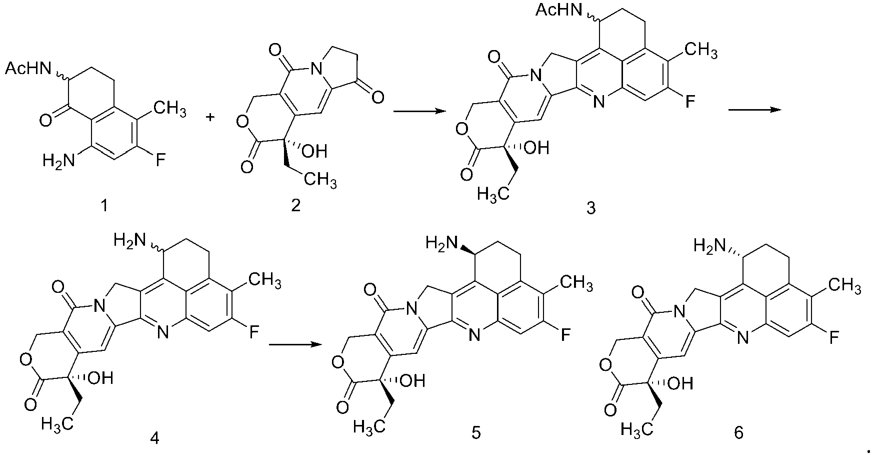

The overall process is achirally selective, with the dr value of the resulting diastereomer 4 being about 1:1. The subsequent recrystallization step serves only to purify the optical product. For this, the single-step recrystallization yield is about 45%, and the overall process yield is less than 41%. For other synthesis references, see CN111065621, CN115197088, and CN115197234.

### SUMMARY

The present disclosure provides a method for preparing a compound of formula I or a salt thereof 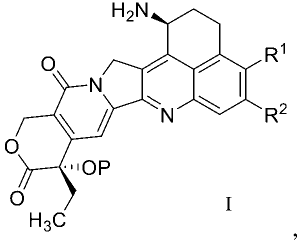 the method comprising a step of reacting a compound of formula D-a under acidic conditions to form the compound of formula I: 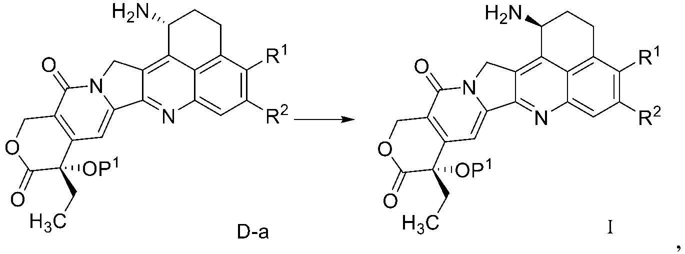 wherein P¹ is a hydroxy protecting group or hydrogen, e.g., hydrogen or tert-butoxycarbonyl; R¹ is selected from the group consisting of hydrogen, halogen, and C₁₋₆ alkyl, e.g., methyl; R² is selected from the group consisting of hydrogen and halogen, e.g., fluorine.

In some embodiments, the acid used in the reaction is selected from the group consisting of a hydrogen chloride (HCl) solution. In some embodiments, the acid used in the reaction is a HCl/1,4-dioxane solution, a HCl/ethanol solution, a HCl/ethyl acetate solution, a HCl/isopropanol solution, or a HCl/isobutanol solution.

In some embodiments, the solvent used in the reaction of the compound of formula D-a is selected from the group consisting of one or more of isobutanol, isopropanol, 3-methyl-2-butanol, ethyl acetate, ethanol, and 1,4-dioxane.

In some other embodiments, the method further comprises a step of reacting a compound of formula A with a compound of formula B to form a compound of formula C: 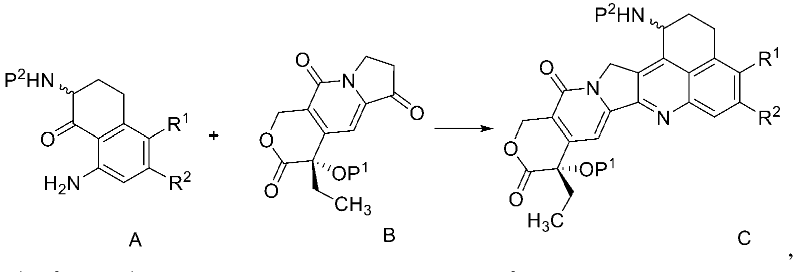 wherein R¹, R², and P¹ are as defined in the foregoing, and P² is selected from the group consisting of an amino protecting group, e.g., acetyl.

The amount (in moles) of the compound of formula B used in this step is not particularly restricted, as long as the amount ensures that the reaction proceeds; preferably, the amount is 0.8-1.2 equivalents (eq.), including 0.8 equivalents, 0.9 equivalents, 1 equivalent, 1.1 equivalents, 1.2 equivalents, or a value between any two of these numbers, based on the compound of formula A.

In some embodiments, the aforementioned compound of formula A is reacted with the aforementioned compound of formula B in the presence of an acidic catalyst. As an example, the acidic catalyst may be pyridinium p-toluenesulfonate or methanesulfonic acid. In some embodiments, the amount (in moles) of the acidic catalyst used in this step is not particularly restricted and is preferably 0.03 to 0.3 equivalents based on the compound of formula A.

In some embodiments, the aforementioned compound of formula A is reacted with the aforementioned compound of formula B in a solvent comprising cresol or phenol. In some embodiments, the compound of formula A is reacted with the compound of formula B in toluene containing o-cresol. Specific reaction conditions/procedures can be found in CN111065621, and relevant contents are incorporated herein for illustrative purposes. In some embodiments, the aforementioned compound of formula A is reacted with the aforementioned compound of formula B in a solvent comprising acetic acid or toluene. In some embodiments, the compound of formula A is reacted with the compound of formula B in toluene containing acetic acid. Faster reaction rates can be observed with the solvent of acetic acid or toluene than with the presence of o-cresol or phenol. Specific reaction conditions/procedures can be found in CN115197088, and relevant contents are incorporated herein for illustrative purposes.

In some embodiments, the temperature at which the aforementioned compound of formula A is reacted with the aforementioned compound of formula B is not restricted, as long as the reaction proceeds; preferably, the temperature is 90-130 °C, including, but not limited to, 90 °C, 100 °C, 110 °C, 120 °C, 130 °C, or any value between two of these numbers. In some embodiments, the temperature at which the aforementioned compound of formula A is reacted with the aforementioned compound of formula B is the thermal reflux temperature of toluene. The reaction time for this step is not restricted, as long as the reaction proceeds; preferably, the reaction time is 16-64 h.

In the preparation method provided in some embodiments, the compound of formula I is 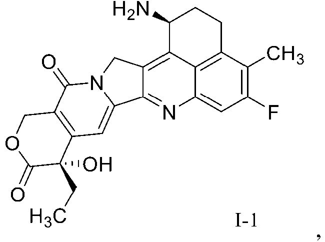 and the method comprises a step of reacting the compound of formula D-1a under acidic conditions to form the compound of formula I-1:

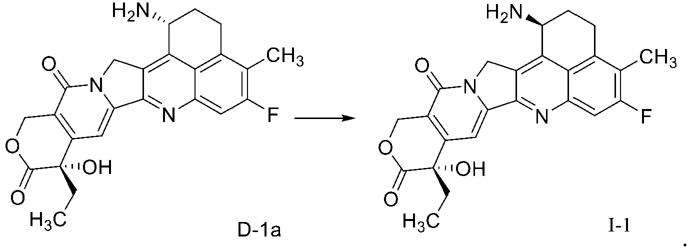

Some embodiments provide a method for preparing a compound of formula I or a salt thereof: 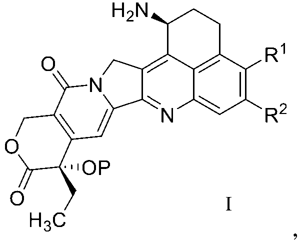 the method comprising a step of enriching a compound of formula D in the presence of an acid to give the compound of formula I, or a step of enriching the compound of formula I in the presence of an acid: 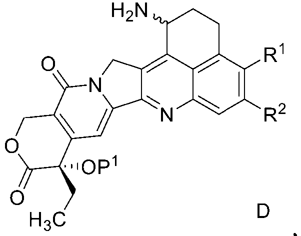 wherein P¹ is a hydroxy protectine group or hydrogen, preferably hydrogen or tert-butoxycarbonyl; R¹ is selected from the group consisting of hydrogen, halogen, and C₁₋₆ alkyl, preferably methyl; R² is selected from the group consisting of hydrogen and halogen, preferably fluorine.

In some embodiments, the compound of formula D comprises a compound of formula D-a and a compound of formula I.

As used herein, "enrich" differs from the crystallization-based separation of diastereomers and is based on the following principle: the product with the target configuration is "enriched" by a dynamic asymmetric conversion process driven by crystallization, e.g., converting the product without the target configuration into the product with the target configuration, under specific conditions, such as the presence of an acid. For example, in some embodiments of the present disclosure, "enrich" refers to the conversion of a compound of formula D-a into a compound of formula I in the presence of an acid, or in some embodiments of the present disclosure, "enrich" refers to the conversion of a compound of formula D into a compound of formula I in the presence of an acid.

In some embodiments, the compound of formula D is a diastereomer mixture, mainly comprising the two diastereomers, compound 5 and compound 6, and the dr value is about 1:1. In some embodiments, the compound of formula D can effectively achieve the conversion of the non-target configuration into the target configuration in the presence of an acid, such as a hydrogen chloride solution, and thereby the enrichment of the compound of formula I. In some embodiments, the dr value of the compound of formula D is increased from 1:1 to about 9:1 in the presence of an acid, such as a hydrogen chloride solution.

In some embodiments, compound 6 is converted into compound 5 (also referred to as the compound of formula I-1) in the presence of an acid: 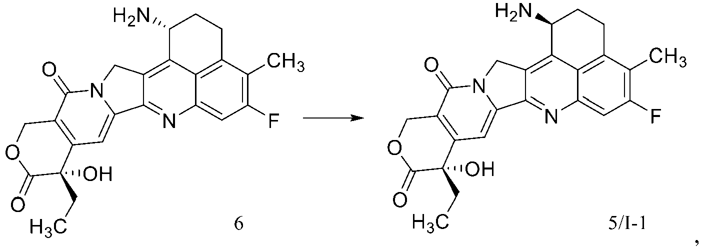 wherein the acid used is selected from the group consisting of a hydrogen chloride solution. In some embodiments, the acid used in the reaction is a HCl/1,4-dioxane solution.

In some embodiments, the method further comprises a step of reacting a compound of formula A-1 with a compound of formula B-1 to form a compound of formula C-1: 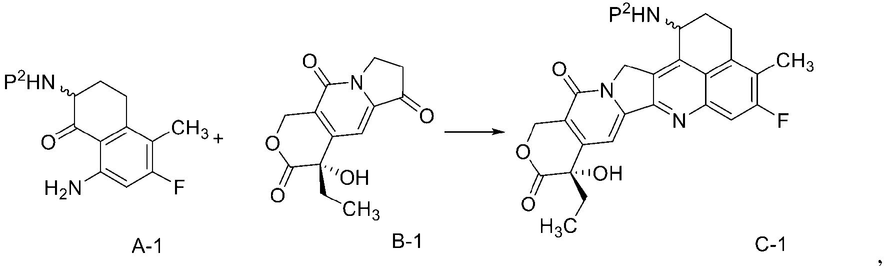 wherein P² is selected from the group consisting of an amino protecting group, preferably acetyl.

In some other embodiments, the method further comprises a step of converting formula C into a compound of formula D:

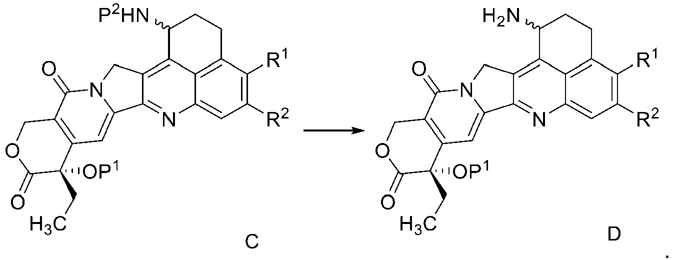

In some embodiments, the compound of formula C is reacted in the presence of an acid, for example, in the presence of methanesulfonic acid and water or in the presence of hydrochloric acid.

In some embodiments, the solvent used in the reaction of the compound of formula C is not particularly restricted, as long as it does not inhibit the reaction; for example, a solvent containing 2-methoxyethanol and ethylcyclohexane is used. In some other embodiments, a solvent of acetic acid or toluene is used in the reaction of the compound of formula C. In some embodiments, an amino protecting group is removed from the compound of formula C in toluene containing acetic acid.

In some embodiments, the reaction temperature at which the aforementioned formula C is converted into the compound of formula D is not restricted, as long as the reaction proceeds; preferably, the reaction temperature is 90-130 °C, including, but not limited to, 90 °C, 100 °C, 110 °C, 120 °C, 130 °C, or any value between two of these numbers. In some embodiments, the reaction temperature at which the aforementioned formula C is converted into the compound of formula D is the thermal reflux temperature of toluene. The reaction time for this step is not restricted, as long as the reaction proceeds; preferably, the reaction time is 16-32 h.

In some embodiments, the method further comprises a step of converting formula C-1 into the compound of formula D-1:

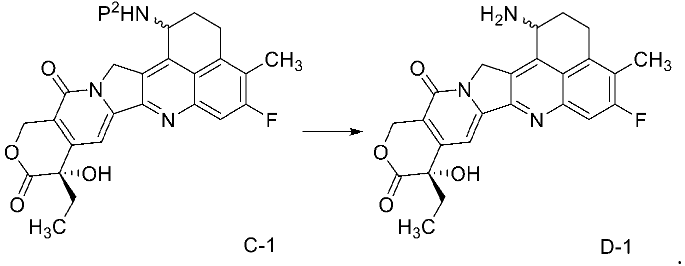

Some embodiments provide a method for preparing a compound of formula I or a salt thereof, the method comprising a step of reacting compound 1 with compound 2 to form compound 3, and a step of removing the amino protecting group, acetyl, from compound 3:

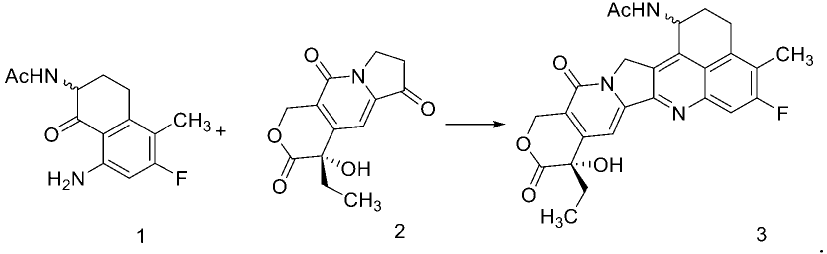

The reaction and/or procedure in this step is the same as the reaction of the compound of formula A with the compound of formula B. In some embodiments, compound 1 is reacted with compound 2 in the presence of an acidic catalyst. As an example, the acidic catalyst may be pyridinium p-toluenesulfonate or methanesulfonic acid. In some embodiments, compound 1 is reacted with compound 2 in the presence of methanesulfonic acid. Further, compound 1 is reacted with compound 2 in toluene containing acetic acid. Specific reaction conditions/procedures can be found in CN115197088, and relevant contents are incorporated herein for illustrative purposes. In another aspect, the present disclosure provides a method for preparing a compound of formula I or a salt thereof, the method comprising reacting a compound of formula A-1 with a compound of formula B-1 to form a compound of formula C-1 and then removing a protecting group from the compound of formula C-1 to form the compound of formula D-1: 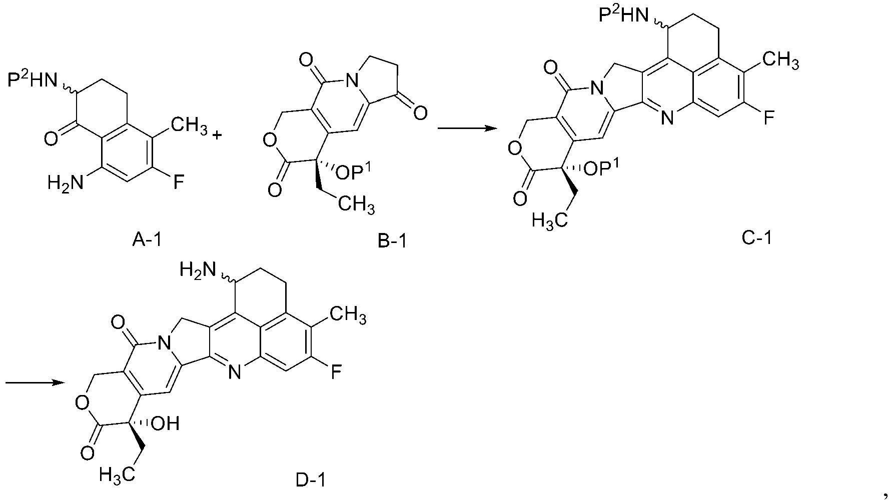 wherein P² is an amino protecting group, e.g., acetyl.

In some embodiments, the method for preparing a compound of formula I or a salt thereof comprises the following steps:
step 1) reacting compound 1 with compound 2 to form compound 3,
step 2) removing an amino protecting group from compound 3 to form compound 4 (also referred to as the compound of formula D-1), and
step 3) enriching compound 5 (also referred to as the compound of formula I-1):

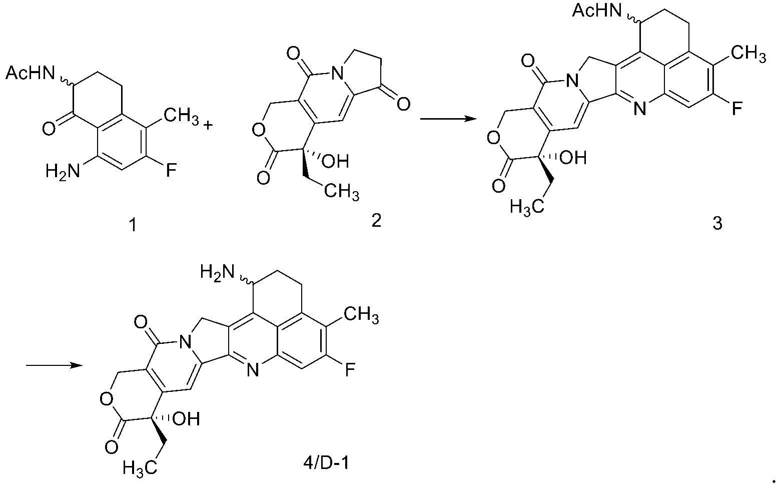

In some embodiments, the step of enriching compound 5 (also referred to as the compound of formula I-1) comprises a step of converting compound 6 into compound 5 (also referred to as the compound of formula I-1) in the presence of an acid:

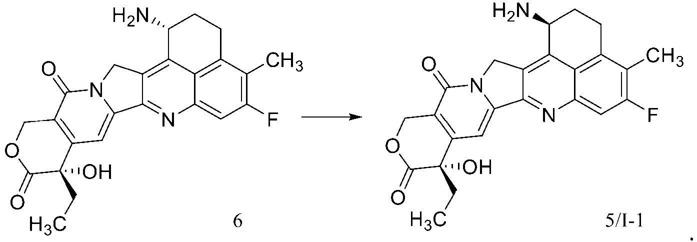

In some embodiments, the acid used in the enrichment is selected from the group consisting of a solution of hydrogen chloride. In some embodiments, the acid used in the enrichment is a HCl/1,4-dioxane solution, a HCl/ethanol solution, a HCl/ethyl acetate solution, a HCl/isopropanol solution, or a HCl/isobutanol solution.

In another aspect, the present disclosure further provides a method for preparing compound DC, the method comprising the aforementioned steps of preparing a compound of formula I: 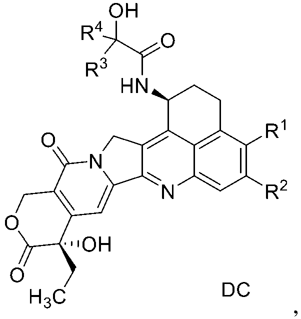 wherein R¹ and R² are as defined in claim 1, and R³ and R⁴ are each independently selected from the group consisting of hydrogen, deuterium, C₁₋₃ alkyl, and C₃₋₆ cycloalkyl, for example, from the group consisting of hydrogen, deuterium, and cyclopropyl.

In some embodiments, the method for preparing compound DC comprises a step of reacting a compound of formula I with compound AA to form a compound of formula DC: 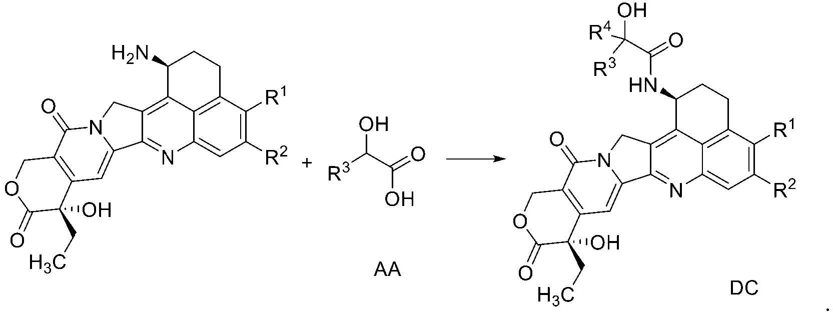 In some embodiments, compound AA is reacted with a compound of formula I in the presence of a basic reagent to form a compound of formula DC. As an example, the basic reagent may be N-methylmorpholine or triethylamine. Specific reaction conditions/procedures can be found in WO2020063676, and relevant contents are incorporated herein for illustrative purposes.

In some embodiments, the compound of formula DC is 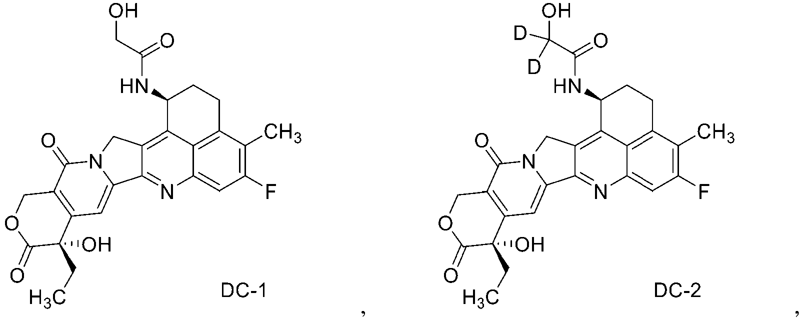 or

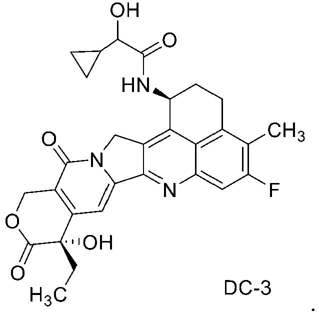

The present disclosure further provides a method for preparing compound DD, the method comprising the aforementioned steps of preparing a compound of formula I, or the aforementioned steps of preparing a compound of formula DC: 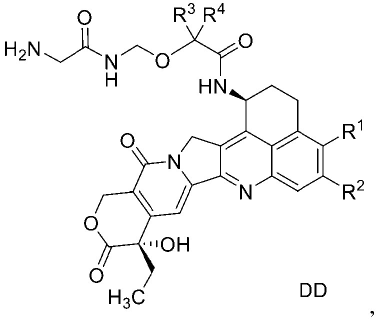 wherein R¹ and R² are as defined in claim 1, and R³ and R⁴ are each independently selected from the group consisting of hydrogen, deuterium, C₁₋₃ alkyl, and C₃₋₆ cycloalkyl, for example, from the group consisting of hydrogen, deuterium, and cyclopropyl.

In some embodiments, the method for preparing compound DD further comprises a step of reacting the compound of formula I with compound BB to form the compound of formula DD: 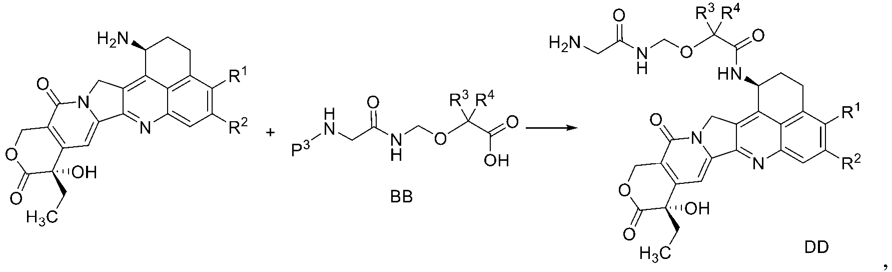 wherein R¹ and R² are as defined in the foregoing, and R³ and R⁴ are each independently selected from the group consisting of hydrogen, deuterium, C₁₋₃ alkyl, and C₃₋₆ cycloalkyl, for example, from the group consisting of hydrogen, deuterium, and cyclopropyl; P³ is an amino protecting group, e.g., Fomc-. In some embodiments, compound B is reacted with a compound of formula I in the presence of a basic reagent to form a compound of formula DD. The base used in the reaction may be N-methylmorpholine or triethylamine. Specific reaction conditions/procedures can be found in WO2020063676, and relevant contents are incorporated herein for illustrative purposes.

In some embodiments, the compound of formula DD is 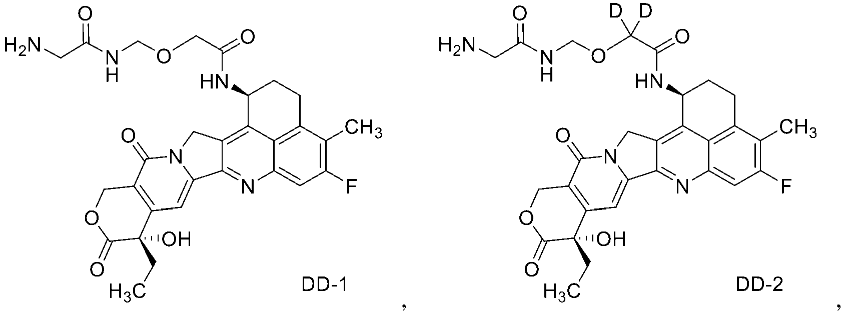 or

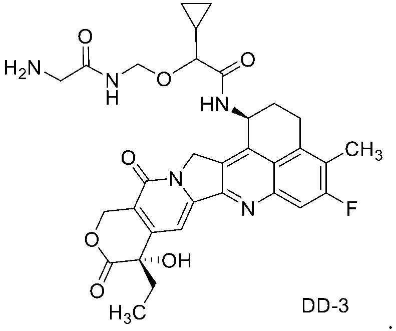

In another aspect, the present disclosure further provides a method for preparing a compound of formula L, the method comprising the aforementioned steps of preparing a compound of formula I, or the aforementioned steps of preparing a compound of formula DC, or the aforementioned steps of preparing a compound of formula DD: 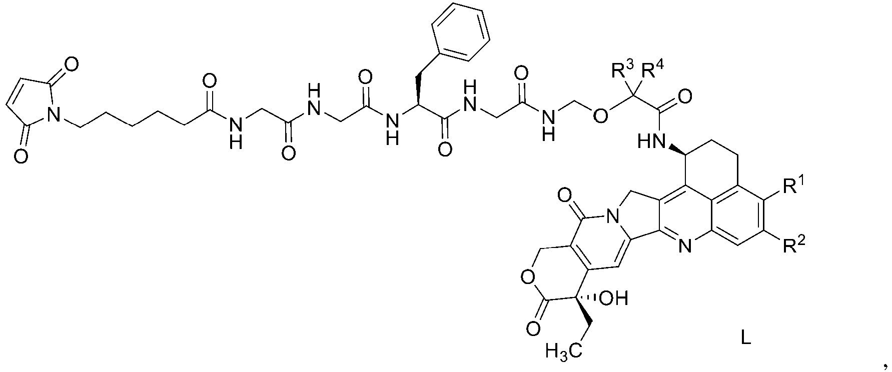 wherein R¹ and R² are as defined in the foregoing, and R³ and R⁴ are each independently selected from the group consisting of hydrogen, deuterium, C₁₋₃ alkyl, and C₃₋₆ cycloalkyl, for example, from the group consisting of hydrogen, deuterium, and cyclopropyl.

In some embodiments, the method for preparing a compound of formula L further comprises a step of reacting the compound of formula I with a compound of formula L-1: 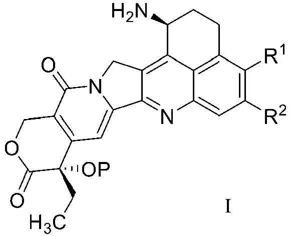 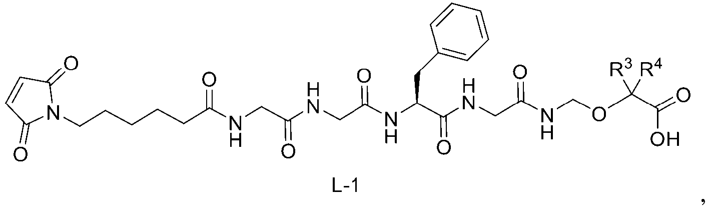 wherein R¹ and R² are as defined in the foregoing, and R³ and R⁴ are each independently selected from the group consisting of hydrogen, deuterium, C₁₋₃ alkyl, and C₃₋₆ cycloalkyl, for example, from the group consisting of hydrogen, deuterium, and cyclopropyl.

In some embodiments, the compound of formula L is 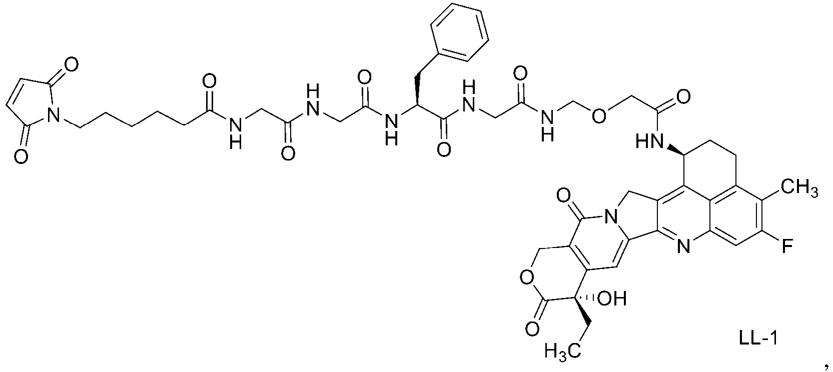 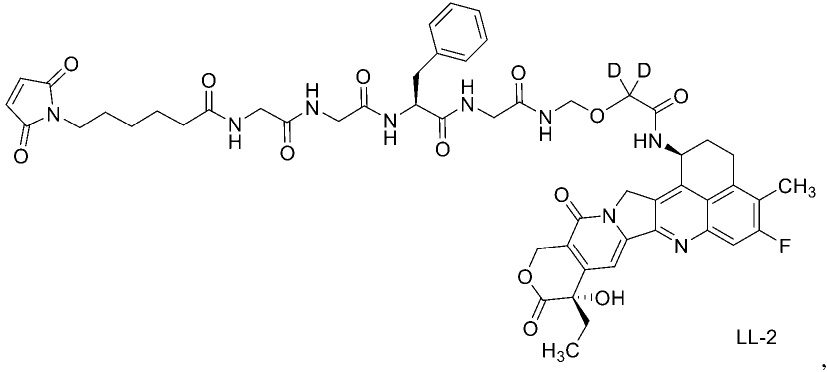 or

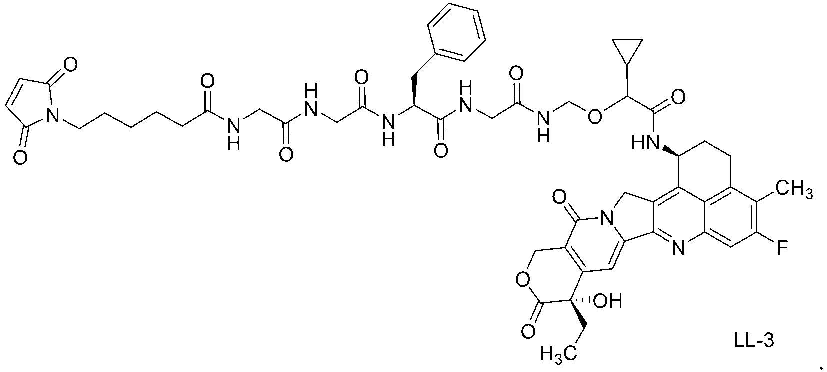

The present disclosure further provides a method for preparing an antibody conjugate, the method comprising the aforementioned steps of preparing a compound of formula I, or the aforementioned steps of preparing a compound of formula DC, or the aforementioned steps of preparing a compound of formula DD, or the aforementioned steps of preparing a compound of formula DD.

The present disclosure further provides a method for preparing an antibody conjugate ADC, the method comprising the aforementioned steps of preparing a compound of formula I, or the aforementioned steps of preparing a compound of formula DC, or the aforementioned steps of preparing a compound of formula DD, or the aforementioned steps of preparing a compound of formula DD, and a step of conjugating formula L to an antibody via a thioether bond:

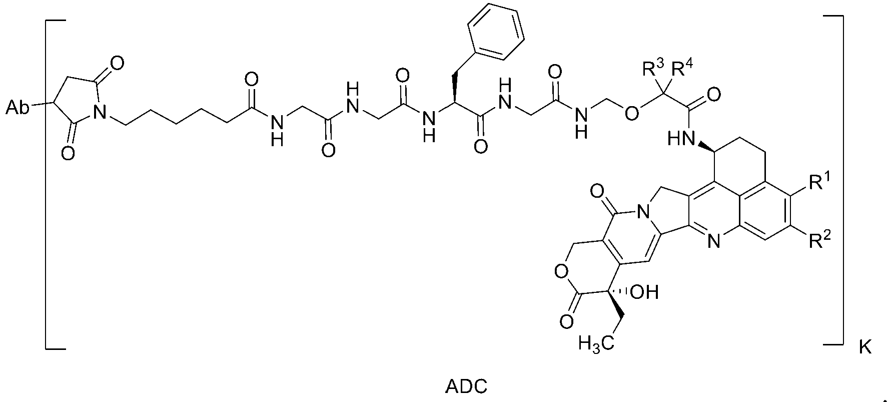

wherein R¹ and R² are as defined in the foregoing; R³ and R⁴ are each independently selected from the group consisting of hydrogen, deuterium, C₁₋₃ alkyl, and C₃₋₆ cycloalkyl, for example, from the group consisting of hydrogen, deuterium, and cyclopropyl; K is 1-10, e.g., 1, 2, 3, 4, or 5; Ab is the antibody or an antigen-binding fragment thereof.

In some embodiments, the antibody or the antigen-binding fragment thereof is selected from the group consisting of an anti-HER2(ErbB2) antibody, an anti-EGFR antibody, an anti-B7-H3 antibody, an anti-c-Met antibody, an anti-HER3(ErbB3) antibody, an anti-HER4(ErbB4) antibody, an anti-CD20 antibody, an anti-CD22 antibody, an anti-CD30 antibody, an anti-CD33 antibody, an anti-CD44 antibody, and an anti-TROP2 antibody.

In some embodiments, the antibody conjugate ADC is 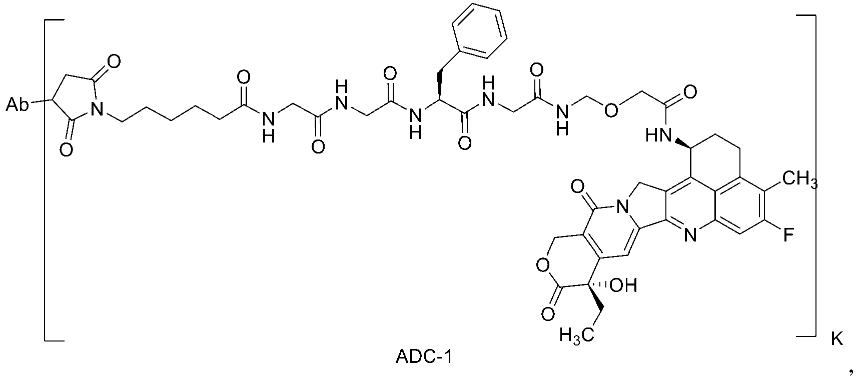 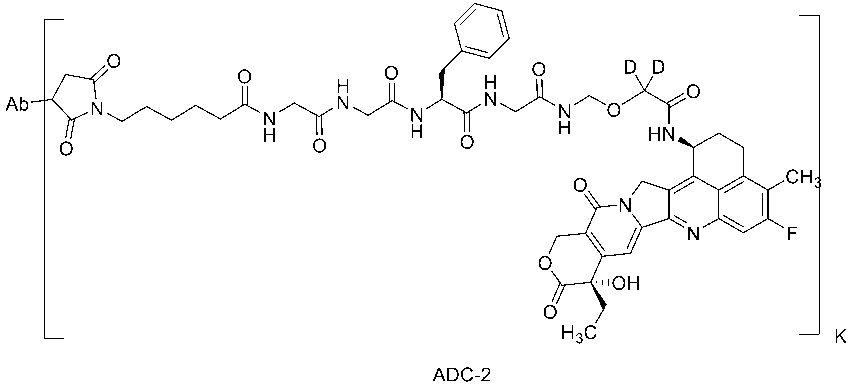 or

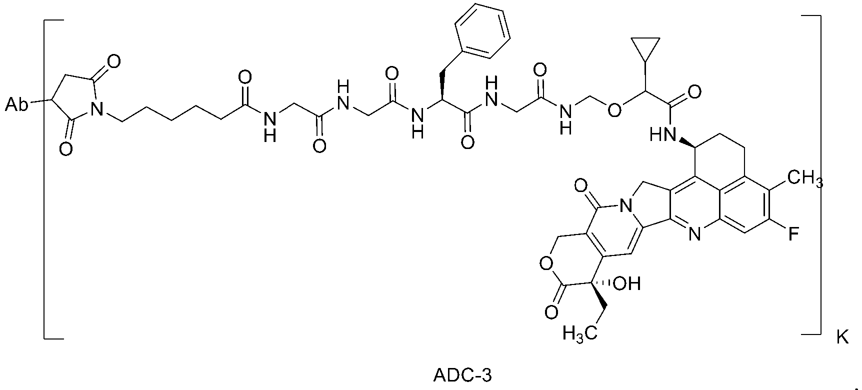

Salts of the compounds/intermediates of the present disclosure include, but are not limited to, addition salts of the compounds/intermediates in free state with acids or bases, and acids used in salt formation include, but are not limited to, hydrochloric acid or methanesulfonic acid. In some embodiments, salts of the compounds/intermediates include, but are not limited to, hydrochloride or mesylate.

The terms "to form" and "convert into" do not specifically refer to a single-step conversion reaction between two substrates; they may refer to a single-step or multi-step reaction between the two substrates. If an intermediate contains a protecting group, the intermediate is subjected to one-step removal of the protecting group and then reacted with the corresponding substrate to give the corresponding target product.

The term "antibody" encompasses a variety of antibody structures, including, but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties), as long as they exhibit the desired antigen-binding activity. "Antigen-binding fragment" encompasses single-chain antibodies (i.e., full-length heavy and light chains); Fab, modified Fab, Fab', modified Fab', F(ab')2, Fv, Fab-Fv, Fab-dsFv, single-domain antibodies (e.g., VH or VL or VHH), scFv, bivalent or trivalent or tetravalent antibodies. Methods for producing or preparing these antigen-binding fragments are well known in the art (see, e.g., Verma et al., 1998, Journal of Immunological Methods, 216, 165-181). The term "antibody-drug conjugate" means that a ligand is linked to a biologically active drug by a stable linker unit. As used herein, "antibody-drug conjugate" (ADC) means that a monoclonal antibody or an antibody fragment is linked to a biologically active toxic drug, such as exatecan or a derivative thereof, by a stable linker unit.

The term "drug loading" may also be represented as the ratio of the number of drug molecules to the number of antibody molecules. The drug loading may range from 1 to 10 cytotoxic drug molecules (D) linked per antibody molecule (Ab). In an embodiment of the present disclosure, the drug loading is represented as DAR or k. Low drug loading can reduce efficacy, while high drug loading can negatively affect the pharmacokinetic profile and toxicity. Illustratively, the drug loading may be an average of 1, 2, 3, 4, 5, 6, 7, 8, 9, 10, or a numerical value between any two of these numerical values, preferably an average of 1-8, or 2-8, or 2-7, or 3-8, or 3-7, or 3-6, or 4-7, or 4-6, or 4-5. The average number of drug molecules per ADC molecule after the coupling reaction can be characterized by conventional methods such as UV/visible spectroscopy, mass spectrometry, an ELISA assay, a mAb molecule size variant assay (CE-SDS), and HPLC. The monoclonal antibody molecular size variant assay (CE-SDS) of the present disclosure may be used for quantitatively determining the purity of a recombinant monoclonal antibody product by adopting capillary electrophoresis-sodium dodecyl sulfate (CE-SDS) ultraviolet assay based on the molecular weight under reduced and nonreduced conditions and according to a capillary electrophoresis method (Chinese Pharmacopoeia).

The numerical values in the present disclosure are instrument measurements and have a certain degree of error. Generally, ±10% is a reasonable margin of error. It is certainly necessary to consider the context in which the numerical values are used, for example, the numerical value of the particle size of the active ingredient after the measurement has a margin of error of no greater than ±10%; the margin of error may be ±9%, ±8%, ±7%, ±6%, ±5%, ±4%, ±3%, ±2%, or ±1%, preferably ±5%.

The pharmaceutically acceptable salts of the compounds described in the present disclosure may be selected from the group consisting of inorganic salts and organic salts, including "acid" addition salts and "base" addition salts. For example, salts formed by acid-base reactions with basic groups (amino groups) are included, and the acid includes organic or inorganic acids.

In the chemical structures of the compounds of the present disclosure, the bond " 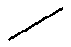 " does not specify a configuration; that is, if chiral isomers exist in the chemical structures, the bond " 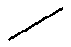 " may be " 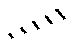 " or " 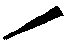 ", or both the configurations " 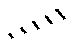 " and " 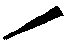 " are included. The bond " 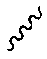 " indicates that chiral isomers exist in the structure, and both the configurations " 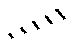 " and " 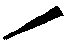 " are included.

As used herein, "antibody conjugate" means that a ligand is linked to a biologically active drug by a stable linker unit. As used herein, "antibody-drug conjugate" means that a monoclonal antibody or an antibody fragment is linked to a biologically active toxic drug, such as exatecan or a derivative thereof, by a stable linker unit.

The term "alkyl" refers to a saturated aliphatic hydrocarbon group, including alkyl groups having 1 to 6 carbon atoms. Non-limiting examples include methyl, ethyl, n-propyl, and isopropyl.

The term "cycloalkyl" refers to a saturated monocyclic or polycyclic hydrocarbon substituent; the cycloalkyl ring contains 3 to 6 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, etc. Polycyclic cycloalkyl groups include spiro-ring, fused-ring, and bridged-ring cycloalkyl groups.

"Halogen" refers to fluorine, chlorine, bromine, or iodine.

As used herein, "protecting group", such as "hydroxy protecting group" and "amino protecting group", is a group known in the art for hydroxy or amino protection; see the amino or hydroxy protecting groups in "Protective Groups in Organic Synthesis", 5Th Ed. T. W. Greene & P. G. M. Wuts. As an example, amino protecting groups include, but are not limited to, acetyl or Fomc.

### DETAILED DESCRIPTION

The present disclosure is further described below with reference to examples. However, these examples do not limit the scope of the present disclosure.

Experimental methods without conditions specified in the examples of the present disclosure were generally conducted under conventional conditions, or conditions recommended by the manufacturers of the starting materials or commercial products. Reagents without specific origins indicated are commercially available conventional reagents.

The structures of the compounds were determined by nuclear magnetic resonance (NMR) spectroscopy or/and mass spectrometry (MS). The NMR shifts (δ) are given in 10-6 (ppm).

The NMR analyses were performed using a Bruker AVANCE-400 nuclear magnetic resonance instrument, with chloroform-D (CDCl₃) as a solvent.

The MS analyses were performed using a Waters Micromass Quattro micro API triple quadrupole mass spectrometer in positive/negative ion scan mode, with the mass scan range being 120-1300.

HPLC conditions (applicable to intermediate G, intermediate H, intermediate H-1, intermediate H-2, intermediate I-1, and intermediate I-2): chromatography column: octadecylsilane bonded silica gel as a filler (Agilent Zorbax Bonus-RP C18, 4.6 mm × 150 mm, 3.5 µm); column temperature: 30 °C; detection wavelength: 240 nm; mobile phases: phosphate buffer as mobile phase A, and methanol as mobile phase B; the retention time of intermediate G was about 17-20 min, the retention times of intermediate H-1 and intermediate I-1 were about 7-8 min, and the retention times of intermediate H-2 and intermediate I-2 were about 9-10 min.

The thin-layer chromatography silica gel plates used were Yantai Huanghai HSGF254 silica gel plates. The silica gel plates used in the thin-layer chromatography (TLC) analyses had a layer thickness of 0.2 mm ± 0.03 mm, and those used in the thin-layer chromatography separation and purification had a layer thickness of 0.4 mm-0.5 mm.

### Example 1:

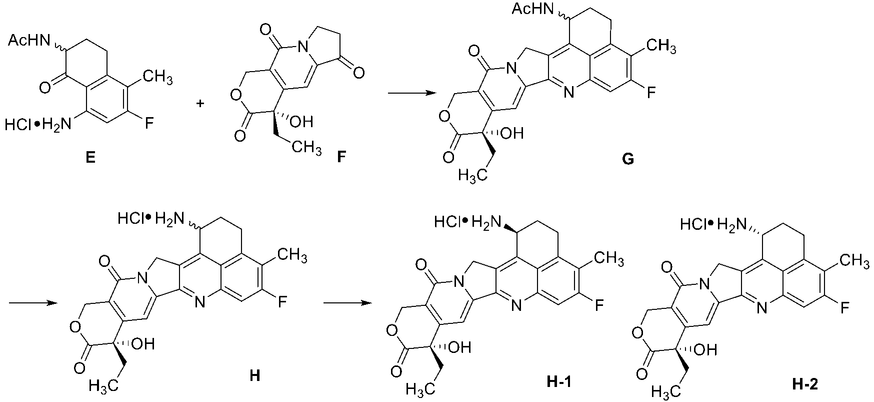

### Step 1:

90 g of intermediate E, 86.7 g of intermediate F, 180 mL of acetic acid, 6.03 g of methanesulfonic acid, and 900 mL of toluene were added to a reaction flask, and the mixture was heated at reflux. After the reaction was complete, 900 mL of toluene was added, and the mixture was cooled to below room temperature, filtered, and dried to give intermediate G.

Step 2: 3.5 L of 8 M hydrochloric acid, 750 mL of anhydrous acetic acid, 1.5 L of toluene, and intermediate G (153 g) were added to a reaction flask and uniformly stirred. The mixture was heated at reflux. After the reaction was complete, the reaction mixture was cooled to room temperature and filtered using a Büchner funnel with 300 g of diatomaceous earth in it. The filter cake was washed with 3 L of a mixed solvent (methanol:purified water = 1:1) and concentrated to give product H (153 g). According to HPLC analysis, the dr value was 52%:48%.

### Step 3:

72 g of the product obtained in step 2, 1080 mL of isobutanol, and 1400 mL of a 1 M HCl/1,4-dioxane solution were added to a reaction flask. The mixture was heated to 60 °C and stirred. After the reaction was complete, the reaction mixture was cooled to room temperature and concentrated under reduced pressure at 45 °C until no liquid significantly dripped, giving a yellow crude product. According to HPLC analysis, the dr value (content ratio) was 90%:10%.

Step 4: A 6 N hydrochloric acid solution (1080 mL) and the yellow crude product obtained in step 3 were added to a reaction flask. The mixture was heated to 45 °C and filtered using a Büchner funnel with 300 g of diatomaceous earth in it. The filter cake was washed with a 6 N hydrochloric acid solution. The filtrate was transferred to a reaction flask and heated to 45 °C, and 2.88 L of methanol was added dropwise. After the dropwise addition, the mixture was cooled to room temperature and filtered under reduced pressure. The filter cake was washed with absolute methanol (50 mL per wash) and dried in a vacuum drying oven at 20-25 °C for 12-15 h to give compound H-1 (55.4 g). According to HPLC analysis, the dr value was 99%:1%. The overall yield of the steps from E to H-1 was 75%.

### Example 2:

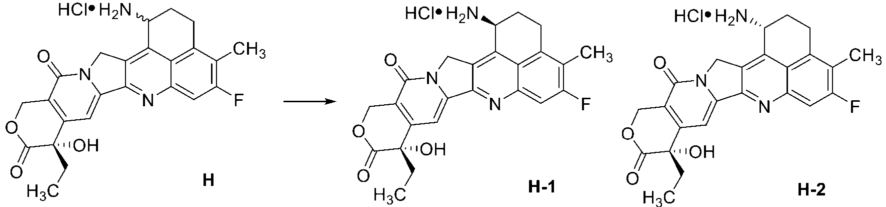

0.5 g of the product obtained in step 2 of Example 1 was added to a reaction flask, 20 volumes (V) of isobutanol solvent (10 mL) was added, and then 10 volumes (V) of a HCl/1,4-dioxane solution (1 M, 5 mL) was slowly added. The mixture was heated to 60 °C, stirred for 20 h, cooled to room temperature, and concentrated. A sample was then taken for HPLC analysis, and the results showed that the content ratio of diastereomer H-1 to diastereomer H-2 was 92.8%/7.2%.

### Example 3:

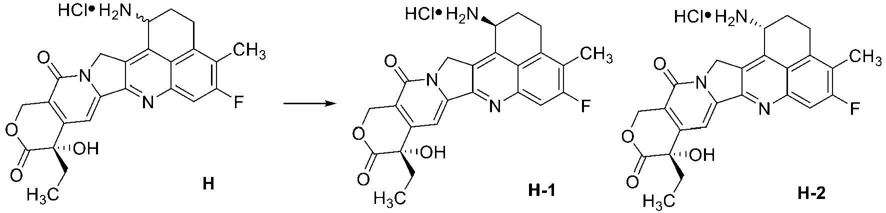

0.5 g of the product obtained in step 2 of Example 1 was added to a reaction flask, 15 volumes (V) of isobutanol solvent (7.5 mL) was added, and then 15 volumes (V) of a HCl/1,4-dioxane solution (1 M, 7.5 mL) was slowly added. The mixture was heated to 60 °C, stirred for 20 h, cooled to room temperature, and concentrated. A sample was then taken for HPLC analysis, and the results showed that the content ratio of diastereomer H-1 to diastereomer H-2 was 92.1%/7.9%.

### Example 4:

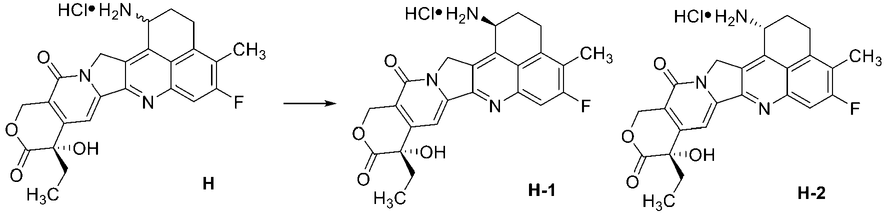

0.5 g of the product obtained in step 2 of Example 1 was added to a reaction flask, 15 volumes (V) of isobutanol solvent (7.5 mL) was added, and then 15 volumes (V) of a HCl/1,4-dioxane solution (2 M, 7.5 mL) was slowly added. The mixture was heated to 60 °C, stirred for 20 h, cooled to room temperature, and concentrated. A sample was then taken for HPLC analysis, and the results showed that the content ratio of diastereomer H-1 to diastereomer H-2 was 92.2%/7.8%.

### Example 5:

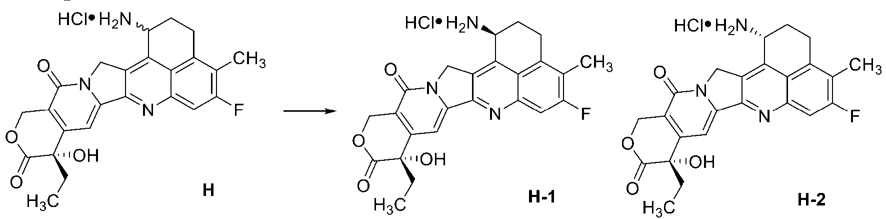

0.5 g of the product obtained in step 2 of Example 1 was added to a reaction flask, and 15 volumes (V) of a HCl/isopropanol solution (4 M, 7.5 mL) was slowly added. The mixture was heated to 60 °C, stirred for 20 h, cooled to room temperature, and concentrated. A sample was then taken for HPLC analysis, and the results showed that the content ratio of diastereomer H-1 to diastereomer H-2 was 89.5%/10.5%.

### Example 6:

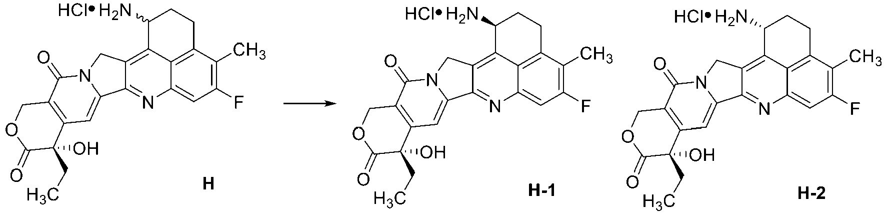

0.5 g of the product obtained in step 2 of Example 1 was added to a reaction flask, 15 volumes (V) of 3-methyl-2-butanol solvent (7.5 mL) was added, and then 15 volumes (V) of a HCl/1,4-dioxane solution (1 M, 7.5 mL) was slowly added. The mixture was heated to 60 °C, stirred for 20 h, cooled to room temperature, and concentrated. A sample was then taken for HPLC analysis, and the results showed that the content ratio of diastereomer H-1 to diastereomer H-2 was 83.2%/16.8%.

### Example 7:

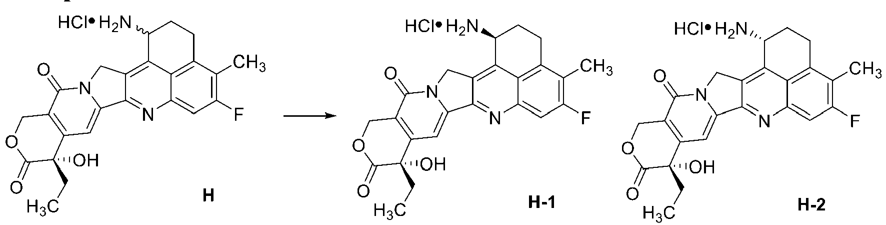

0.5 g of the product obtained in step 2 of Example 1 was added to a reaction flask, 15 volumes (V) of isobutanol solvent (7.5 mL) was added, and then 15 volumes (V) of a HCl/ethyl acetate (4 M, 7.5 mL) was slowly added. The mixture was heated to 60 °C, stirred for 20 h, cooled to room temperature, and concentrated. A sample was then taken for HPLC analysis, and the results showed that the content ratio of diastereomer H-1 to diastereomer H-2 was 84.8%/15.2%.

### Example 8:

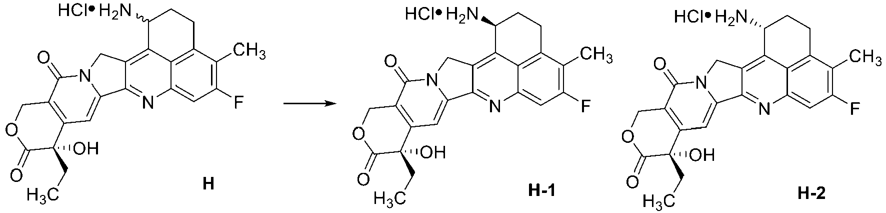

0.5 g of the product obtained in step 2 of Example 1 was added to a reaction flask, 15 volumes (V) of isobutanol solvent (7.5 mL) was added, and then 15 volumes (V) of a HCl/ethanol solution (4 M, 7.5 mL) was slowly added. The mixture was heated to 60 °C, stirred for 20 h, cooled to room temperature, and concentrated. A sample was then taken for HPLC analysis, and the results showed that the content ratio of diastereomer H-1 to diastereomer H-2 was 85.0%/15.0%.

### Example 9:

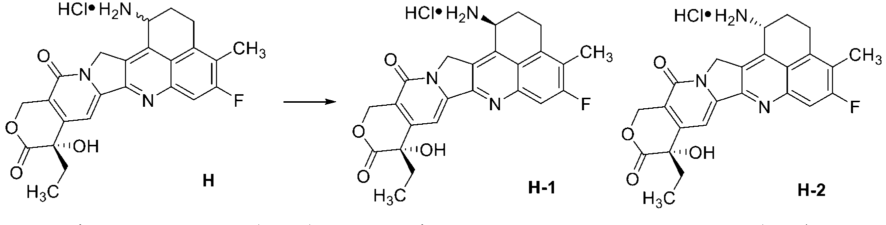

0.5 g of the product obtained in step 2 of Example 1 was added to a reaction flask, 15 volumes (V) of isobutanol solvent (7.5 mL) was added, and then 5 volumes (V) of hydrobromic acid (content ≥ 40%, 2.5 mL) was slowly added. The mixture was heated to 60 °C, stirred for 20 h, cooled to room temperature, and concentrated. A sample was then taken for HPLC analysis, and the results showed that the content ratio of diastereomer H-1 to diastereomer H-2 was 62.2%/37.8%.

### Example 10:

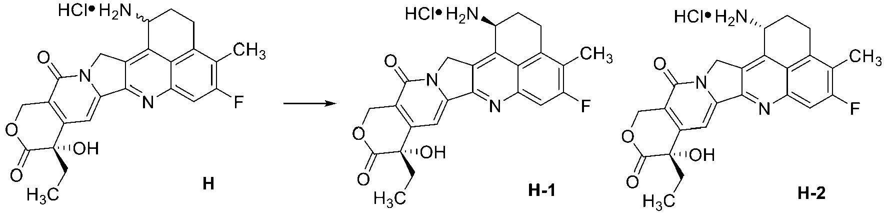

0.5 g of the product obtained in step 2 of Example 1 was added to a reaction flask, 15 volumes (V) of isobutanol solvent (7.5 mL) was added, and then 5 volumes (V) of concentrated sulfuric acid (2.5 mL) was slowly added. The mixture was heated to 60 °C, stirred for 20 h, cooled to room temperature, and concentrated. A sample was then taken for HPLC analysis, and the results showed that the content ratio of diastereomer H-1 to diastereomer H-2 was 52.7%/47.3%.

### Example 11:

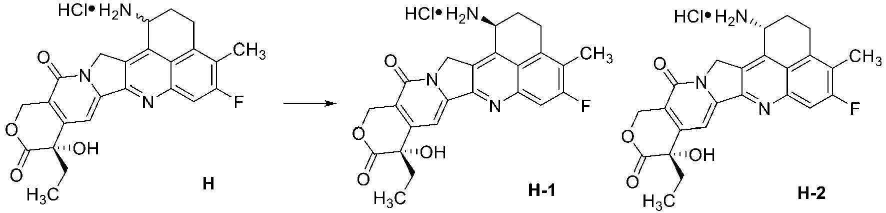

0.5 g of the product obtained in step 2 of Example 1 was added to a reaction flask, 15 volumes (V) of isobutanol solvent (7.5 mL) was added, and then 5 volumes (V) of perchloric acid (2.5 mL) was slowly added. The mixture was heated to 60 °C, stirred for 20 h, cooled to room temperature, and concentrated. A sample was then taken for HPLC analysis, and the results showed that the content ratio of diastereomer H-1 to diastereomer H-2 was 52.5%/47.5%.

### Example 12:

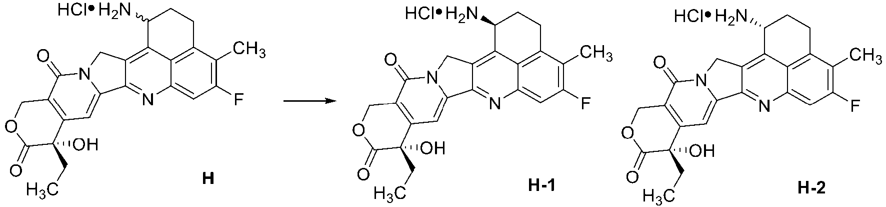

0.5 g of the product obtained in step 2 of Example 1 was added to a reaction flask, and 2.7 mL of methanesulfonic acid (5.4 V) and 0.6 mL of purified water (1.2 V) were added. The mixture was heated to 45 °C and stirred for 30 min, and 10 mL of methanol (18 V) was added dropwise. After the dropwise addition, the mixture was stirred at that temperature for 3 h and naturally cooled to 25 °C. A sample was taken for HPLC analysis, and the results showed that the content ratio of diastereomer H-1 to diastereomer H-2 was 51.5%/48.5%.

### Example 13:

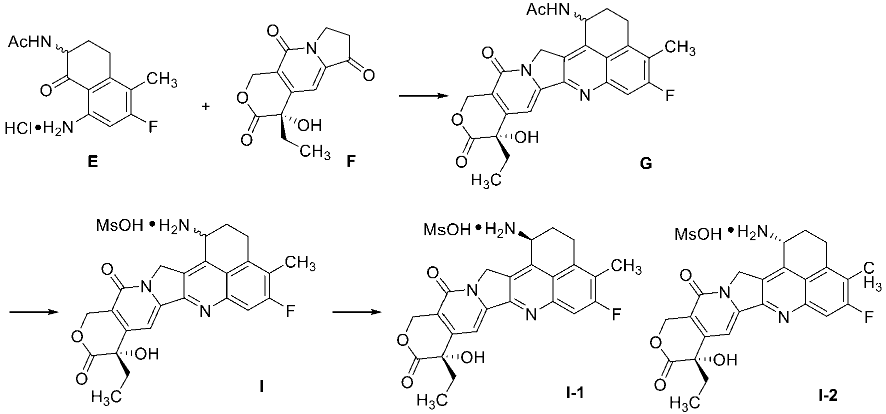

### Step 1:

155.5 g of intermediate E, 150.0 g of intermediate F, and 2.8 L of toluene were added to a reaction flask and stirred, and 10.5 g of methanesulfonic acid and 622 mL of acetic acid were added. The mixture was refluxed. After the reaction was complete, the reaction mixture was cooled and concentrated until almost no liquid dripped, giving intermediate G.

### Step 2:

The intermediate G obtained in the previous step, 1.6 L of toluene, 3.110 L of purified water, and 1.555 L of methanesulfonic acid were added to a reaction flask and stirred. The mixture was refluxed. After the reaction was complete, the reaction mixture was cooled to 60-65 °C and filtered (with diatomaceous earth as a filter aid). The filter cake was washed with 2 × 1.0 L of a mixture of methanol and water (1:1 by volume), and the filtrate and the wash filtrates were combined. A sample was taken for HPLC analysis, and the results showed that the content ratio of diastereomer I-1 to diastereomer I-2 was 50.2%/49.8%.

### Step 3:

The filtrate was concentrated under reduced pressure until no liquid dripped. The concentrate was transferred to a reaction flask, stirred, and heated to 45 °C, 5.597 L of methanol was added dropwise, and 311 mL of purified water was then added dropwise. The mixture was cooled to room temperature and then filtered. The filter cake was washed with 2 L of methanol and dried in a drying oven at a temperature of 20-25 °C for 12-15 h to give intermediate I-1 (115.0 g). According to HPLC analysis, the dr value was 97%:3%. The overall yield of the three steps (from compound E to compound I-1) was 39.0%.

## Claims

1. A method for preparing a compound of formula I or a salt thereof 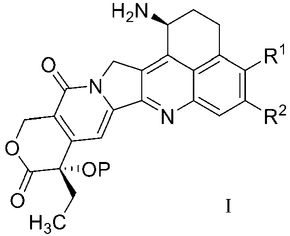 , comprising a step of reacting a compound of formula D-a under acidic conditions to form the compound of formula I, 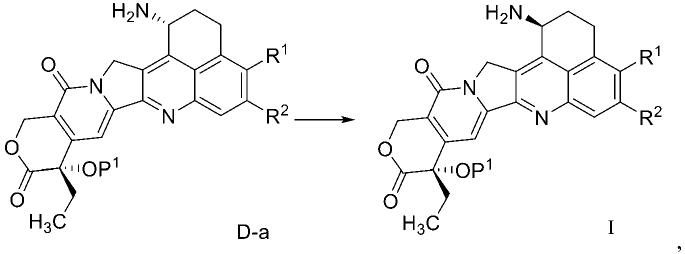 wherein P¹ is a hydroxy protecting group or hydrogen, preferably hydrogen or tert-butoxycarbonyl; R¹ is selected from the group consisting of hydrogen, halogen, and C₁₋₆ alkyl, preferably from the group consisting of methyl; R² is selected from the group consisting of hydrogen and halogen, preferably from the group consisting of fluorine.

2. The method according to claim 1, wherein the acid is selected from the group consisting of a hydrogen chloride solution.

3. The method according to claim 1 or 2, wherein a solution used in the reaction is selected from the group consisting of one or more of isobutanol, isopropanol, 3-methyl-2-butanol, ethyl acetate, ethanol, and 1,4-dioxane.

4. The method according to any one of claims 1-3, further comprising a step of reacting a compound of formula A with a compound of formula B to form a compound of formula C, 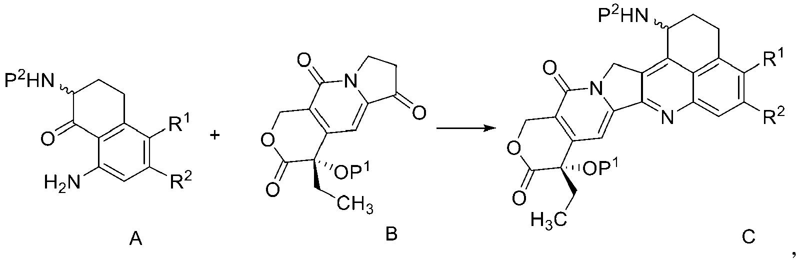 wherein R¹, R², and P¹ are as defined in claim 1, and P² is selected from the group consisting of an amino protecting group, preferably from the group consisting of acetyl.

5. The method according to claims 1-4, wherein the compound of formula I is 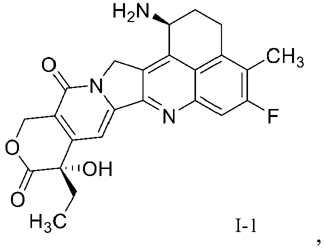 and the method comprises a step of reacting the compound of formula D-1a under acidic conditions to form the compound of formula I-1,

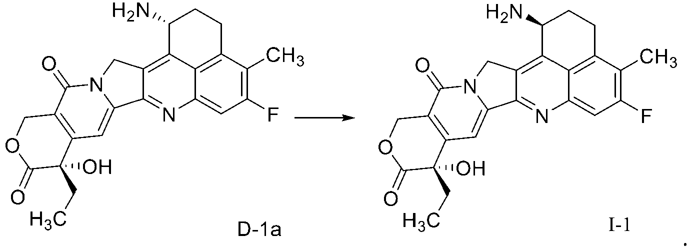

6. A method for preparing a compound of formula I or a salt thereof, 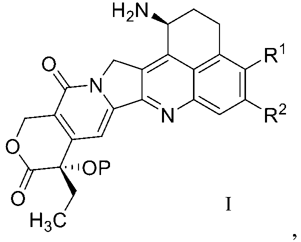 comprising a step of enriching the compound of formula I using a compound of formula D in the presence of an acid, 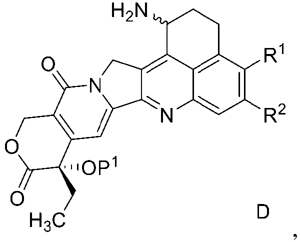 wherein P¹ is a hydroxy protecting group or hydrogen, preferably hydrogen or tert-butoxycarbonyl; R¹ is selected from the group consisting of hydrogen, halogen, and C₁₋₆ alkyl, preferably from the group consisting of methyl; R² is selected from the group consisting of hydrogen and halogen, preferably from the group consisting of fluorine.

7. The method according to any one of claims 1-6, comprising reacting a compound of formula A-1 with a compound of formula B-1 to form a compound of formula C-1, and subsequently removing a protecting group from the compound of formula C-1 to form a compound of formula D-1, 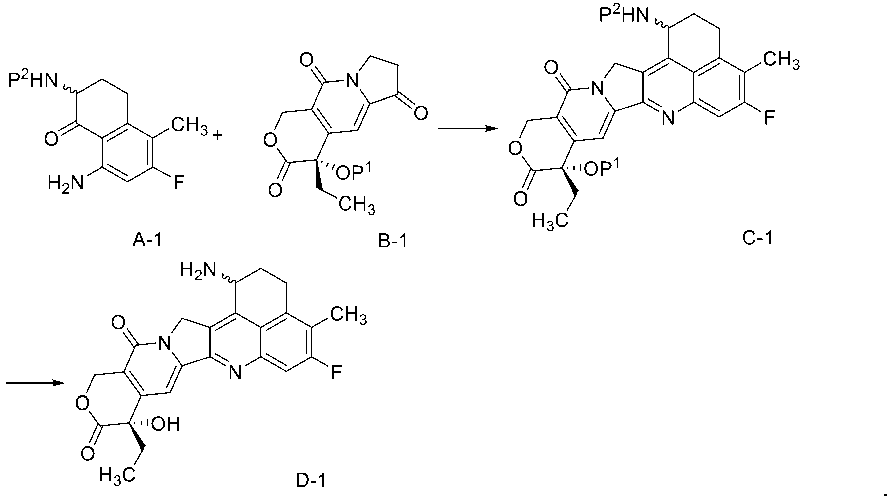 wherein P¹ is a hydroxy protecting group or hydrogen, preferably hydrogen or tert-butoxycarbonyl; P² is an amino protecting group, preferably acetyl.

8. A method for preparing a compound DC, comprising the steps of the method according to any one of claims 1-7, 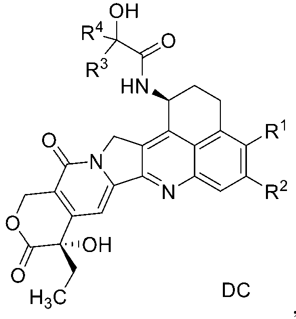 wherein R¹ and R² are as defined in claim 1, and R³ and R⁴ are each independently selected from the group consisting of hydrogen, deuterium, C₁₋₃ alkyl, and C₃₋₆ cycloalkyl, for example, from the group consisting of hydrogen, deuterium, and cyclopropyl.

9. A method for preparing a compound DD, comprising the steps of the method according to any one of claims 1-8, 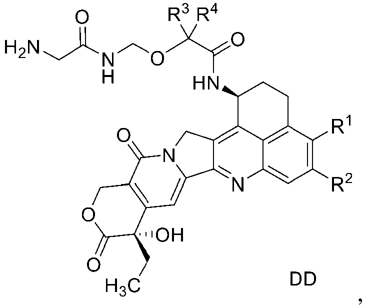 wherein R¹ and R² are as defined in claim 1, and R³ and R⁴ are each independently selected from the group consisting of hydrogen, deuterium, C₁₋₃ alkyl, and C₃₋₆ cycloalkyl, for example, from the group consisting of hydrogen, deuterium, and cyclopropyl.

10. A method for preparing a compound of formula L, 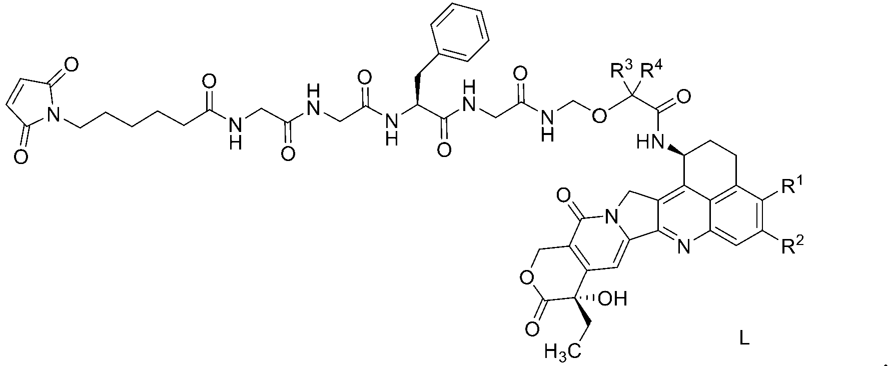 comprising the steps of the method according to any one of claims 1-8, and a step of reacting a compound of formula I with a compound of formula L-1, 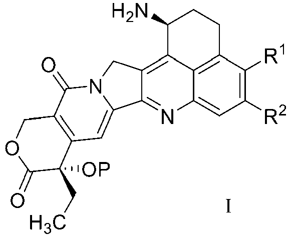 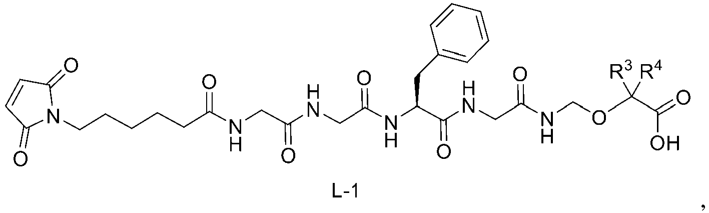 wherein R¹ and R² are as defined in claim 1, and R³ and R⁴ are each independently selected from the group consisting of hydrogen, deuterium, C₁₋₃ alkyl, and C₃₋₆ cycloalkyl, for example, from the group consisting of hydrogen, deuterium, and cyclopropyl.

11. A method for preparing an antibody conjugate, comprising the steps of the method according to any one of claims 1-10.

12. A method for preparing an antibody conjugate ADC, comprising the steps of the method according to any one of claims 1-10 and a step of conjugating formula L with an antibody via a thioether bond, 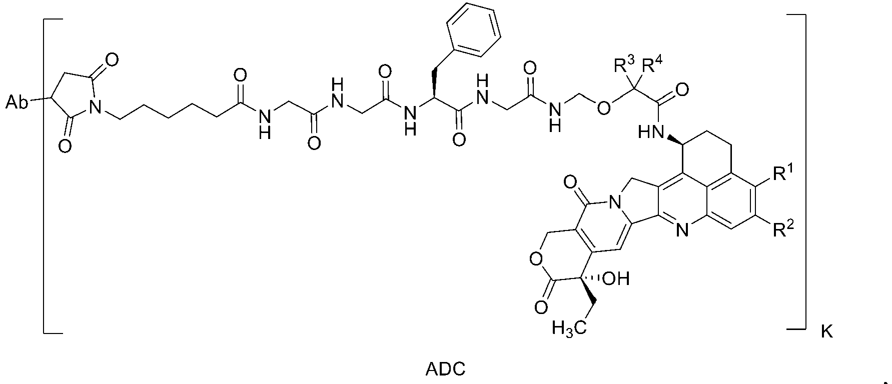 wherein R¹ and R² are as defined in claim 1; R³ and R⁴ are each independently selected from the group consisting of hydrogen, deuterium, C₁₋₃ alkyl, and C₃₋₆ cycloalkyl, for example, from the group consisting of hydrogen, deuterium, and cyclopropyl; K is 1-10; Ab is the antibody or an antigen-binding fragment thereof.

13. The method according to claim 12, wherein the antibody is an anti-HER2 antibody, an anti-HER3 antibody, or an anti-TROP2 antibody.
